# EUROPÄISCHE PATENTSCHRIFT

(11) **EP 1 471 883 B1**
(45) Veröffentlichungstag und Bekanntmachung des Hinweises auf die Patenterteilung: **14.12.2005**
(21) Anmeldenummer: 03711876.7
(22) Anmeldetag: 07.02.2003
(51) Int. Cl.: A61K 9/00

(54) **VERFAHREN ZUR HERSTELLUNG WASSERDISPERGIERBARER TROCKENPULVER VON IN WASSER SCHWER L SLICHEN VERBINDUNGEN**
METHOD FOR PRODUCING WATER DISPERSIBLE DRY POWDERS FROM POORLY SOLUBLE COMPOUNDS
PROCEDE DE PRODUCTION DE POUDRES SECHES DISPERSIBLES DANS L'EAU A PARTIR DE COMPOSES DIFFICILEMENT SOLUBLES DANS L'EAU

(30) Priorität: 08.02.2002 DE 10205362
(43) Veröffentlichungstag der Anmeldung: 03.11.2004
(73) Patentinhaber: Abbott GmbH & Co. KG, 65205 Wiesbaden (DE)
(72) Erfinder: ROSENBERG, Jörg, 67158 Ellerstadt (DE); HANTKE, Thomas, 68165 Mannheim (DE); BREITENBACH, Jörg, 68199 Mannheim (DE)
(74) Vertreter: Reitstötter - Kinzebach
(86) Internationale Anmeldenummer: PCT/EP2003/001244
(87) Internationale Veröffentlichungsnummer: WO 2003/066019

(56) Entgegenhaltungen:
- EP-A- 1 306 127
- DE-A- 19 925 184
- US-A- 5 948 441
- US-A1- 2001 033 868

## Beschreibung

Die vorliegende Erfindung betrifft ein Verfahren zur Herstellung wasserdispergierbarer Trockenpulver von Verbindungen, die in Wasser schwerlöslich oder unlöslich sind und Zubereitungen auf der Basis derartiger wasserdispergierbarer Trockenpulver.

Zahlreiche Verbindungen sind in Wasser schwerlöslich oder unlöslich, sollen aber dennoch in einem wässrigen Medium zur Anwendung kommen. Beispiele hierfür sind bestimmte Arzneimittelwirkstoffe, Nahrungsmittelzusatzstoffe und Kosmetikabestandteile. Es müssen daher Maßnahmen gefunden werden, derartige Verbindungen in wässrigen Systemen ausreichend gut zu lösen, da ansonsten ihre Wirksamkeit stark beeinträchtigt ist. In Wasser schwerlösliche Arzneimittelwirkstoffe werden nach oraler Gabe im Gastrointestinaltrakt ungenügend resorbiert, und bei Farbstoffen, z.B. bei Carotinoiden zum Färben von Lebens- und Futtermitteln, wird nur eine geringe Farbausbeute erzielt. Zur Verbesserung der Solubilisierung der Verbindungen in wässrigen Medien sind bereits verschiedene Möglichkeiten bekannt, z.B. die Verringerung der Partikelgröße der schwerlöslichen Stoffe.

Um Eigenschaften zu erzielen, z.B. Resorptions- oder Färbeeigenschaften, die dem Idealzustand molekularer Dispersion der schwerlöslichen Verbindungen möglichst nahe kommen, müssen die schwerlöslichen Verbindungen in dem wässrigen Medium möglichst fein verteilt werden. Wünschenswert ist dabei eine Partikelgröße von kleiner 1 µm. Durch Vermahlung sind derartige Partikelgrößen entweder überhaupt nicht oder nur unter Schädigung der Verbindungen erreichbar. Bei Carotinoiden wurde versucht, sie unter Verwendung eines wasserlöslichen Lösungsvermittlers zunächst zu lösen und dann durch Verdünnen mit Wasser feinkristallin auszufällen. Dies scheiterte jedoch bisher an der zu geringen Löslichkeit der Carotinoide in solchen Lösungsmitteln.

Eine andere Möglichkeit ist der Zusatz von Solubilisierungs-Hilfsstoffen. Geeignete Solubilisierungs-Hilfsstoffe sind z.B. Tenside, Alkohole, Ether, Ester, etc., für Arzneimittel insbesondere die in den internationalen Arzneibüchern monographierten Solubilisatoren. Mit solchen Solubilisatoren gelingt in vielen Fällen eine micellare Solubilisierung, d.h. die schwerlösliche Verbindung wird an Tensidmicellen angelagert oder in sie eingelagert. In vielen Fällen müssen aber recht große Mengen dieser Solubilisatoren für die schwerlöslichen Wirkstoffe eingesetzt werden. Dies kann bei Arzneimitteln unerwünschte Nebenwirkungen nach oraler Gabe solcher Wirkstoff-Präparationen verursachen.

Eine weitere Möglichkeit, schwerlösliche Verbindungen in optimal nutzbare Form zu bringen, besteht darin, die betreffende Verbindung in Wasser kolloidal zu lösen. In diesem Fall wird die Verbindung in kolloidale Aggregate eingebaut, die aus sogenannten Schutzkolloiden in Wasser hergestellt werden können. Solche Schutzkolloide sind z.B. Gelatine und/oder Casein.

Aus Chimia 21, 329 (1967) sowie DE-AS 12 11 911 und DE-OS 25 34 091 sind Verfahren bekannt, bei denen man den Wirkstoff in einem mit Wasser nicht mischbaren Lösungsmittel, vorzugsweise einem Chlorkohlenwasserstoff, löst, die Lösung durch Homogenisieren in einer Gelatine-Zucker-Lösung emulgiert und aus der Emulsion schließlich das Lösungsmittel abzieht, wobei der Wirkstoff in feinkristalliner Form freigesetzt wird. Aus der erhaltenen Suspension kann durch Entwässern ein feinverteiltes Pulver gewonnen werden. Die Verwendung chlorierter Kohlenwasserstoffe stellt jedoch einen schwerwiegenden Nachteil dieses Verfahrens dar.

Andere Verfahren zur Herstellung eines Präparates mit Feinverteilung der Wirkstoffe sind das Aufbringen der Wirkstoffe auf Trägermaterialien wie Stärke, Pectin oder Trockenmilchpulver, wobei z.B. eine Lösung des Wirkstoffs in Öl gemäß DE-PS 642 307 oder Chloroform gemäß DE-PS 361 637 und CH-PS 304 023 auf die Trägermaterialien aufgesprüht wird. Die erhaltenen Präparate sind jedoch nicht universell in wässrigen Medien dispergierbar und unzulänglich lagerstabil.

In Chimia 21, 329 (1967) und in FR-PS 1 056 114, sowie in US-PS 2,650,895 sind Verfahren beschrieben, bei denen Wirkstoffe in Form ihrer Öllösungen in Kolloide, wie Gelatine, emulsionsartig eingebettet werden. Die Wirkstoffkonzentrationen in den so hergestellten Präparaten sind jedoch wegen der geringen Öllöslichkeit der Wirkstoffe gering.

Weiter sind eine Reihe von Verfahren bekannt, bei denen zunächst eine feinteilige Dispersion der schwerlöslichen Stoffe in einem wässrigen Medium hergestellt wird. Diese Dispersion wird dann durch Entfernen des Mediums in ein feinteiliges Trockenpulver der Stoffe überführt, siehe WO 94/01090, WO 93/10768, EP 239949, EP 425892, DE 37 42 473, etc. Dementsprechend offenbart auch die EP 0 065 193 A2 ein Verfahren zur Herstellung von pulverförmigen Carotinoid- und Retinoid-Präparaten, bei dem man die schwerlösliche Verbindung in einem flüchtigen, mit Wasser mischbaren, organischen Lösungsmittel bei erhöhter Temperatur schnell löst, aus der erhaltenen molekulardispersen Lösung sofort durch schnelles Mischen mit einer wässrigen Lösung eines quellbaren Kolloids die schwerlösliche Verbindung in kolloid-disperser Form ausfällt und die erhaltene Dispersion von dem Lösungsmittel und dem Dispergiermedium befreit.

Die DE 37 02 030 A1 offenbart ein Verfahren zur Herstellung von pulverförmigen wasserdispergierbaren Carotinoid-Zubereitungen, in denen das Carotinoid in einem eßbaren Öl gelöst und die Öllösung in Form kleiner Tröpfchen vorliegt. Hier wird das Carotinoid in einem flüchtigen, mit Wasser mischbaren, organischen Lösungsmittel bei erhöhter Temperatur gemeinsam mit der 1,5- bis 20-fachen Gewichtsmenge, bezogen auf das Carotinoid eines eßbaren Öls, sowie einem Emulgator, schnell gelöst, dann aus der erhaltenen molekulardispersen Lösung durch sofortiges Mischen mit einer wässrigen Lösung eines Schutzkolloids eine Zweiphasen-Mischung gebildet, in der das Öl als mikrodisperse Phase mit darin gelöstem Carotinoid vorliegt. Die pulverförmige Carotinoid-Zubereitung, die nach Entfernen von Lösungsmittel und Wasser erhalten wird, enthält das Carotinoid in dem eßbaren Öl gelöst, und die Öllösung ist in Form kleiner Tröpfchen in der pulverförmigen Schutzkolloid-Matrix dispergiert.

Weitere Verfahren wie die Verfahren gemäß EP 0 065 193 A2 und DE 37 02 030 A1 führen zu redispergierbaren Trockenpulvern, haben aber auch einige Nachteile. Die gebildeten kolloidalen Lösungen sind sehr verdünnt, d.h. typische Feststoffkonzentrationen dieser Kolloidlösungen liegen im Bereich von 0,5 bis maximal 3 Gew.-%. Dies bedeutet, dass zur Herstellung des für ein Medikament oder ein sonstiges Produkt, z.B. einen Lebensmittelfarbstoff, benötigten Pulvers eine erhebliche Menge Lösungsmittel, und zwar im Wesentlichen Wasser, entfernt werden muß. Das zur Herstellung derartiger Pulver geeignetste Trocknungsverfahren ist die Sprühtrocknung, die im Labormaßstab gut durchführbar ist. Eine auch nur annähernd wirtschaftliche Herstellung im Produktionsmaßstab ist allerdings nicht möglich. Bereits zur Herstellung von 100 kg sprühgetrockneten Pulvers müssen im Fall einer Kolloidlösung, die einen Gesamt-Feststoffgehalt von 3 Gew.-% besitzt über 3000 1 Kolloidlösung sprühgetrocknet werden.

Ein weiterer Nachteil ist, dass die in den Kolloidlösungen vorliegenden Partikel bei einer Lagerung der Lösungen zum Agglomerieren neigen, wodurch immer größere Partikel entstehen, die schließlich sedimentieren. Dies bedeutet, dass die Kolloidlösungen schnell, ohne Zwischenlagerung, getrocknet werden müssen. Eine "on-line"-Variante, bei der Kolloidlösungen mit einem Feststoffgehalt von 1 bis 3 Gew.-% unmittelbar nach ihrer Herstellung sofort getrocknet werden, erfordert bei dem bevorzugten Verfahren der Sprühtrocknung jedoch eine sehr große und damit unwirtschaftliche Sprühkapazität.

Ein zusätzlicher Nachteil besteht darin, dass die typischerweise verwendeten Schutzkolloide Naturstoffe oder Naturstoff-Derivate sind, wie z.B. Casein oder Gelatine, deren wässrige Lösungen einem schnellen mikrobiellen Befall unterliegen. Auch aus diesem Grund ist eine Lagerung der kolloidalen Lösungen der schwerlöslichen Verbindungen nicht über einen längeren Zeitraum möglich, außer gegebenenfalls bei aufwendigem keimfreiem Arbeiten und/oder bei Zugabe von Konservierungsmitteln zur Keimreduktion.

Es hat sich gezeigt, dass herkömmliche Verfahren zur Erhöhung des Feststoffgehalts einer Dispersion Nachteile aufweisen. An dem Verfahren der Zentrifugation beispielsweise ist nachteilig, dass die geringe Partikelkonzentration und die geringe Partikelgröße bei der vorliegenden Erfindung lange Prozesszeiten und hohe Zentrifugalkräfte erfordern.

Die konventionelle (Dia-) Filtration ist unbrauchbar, da im Falle der vorliegenden Erfindung, bei der die schwerlöslichen Verbindungen in kolloidaler Dispersion vorliegen, eine über die Prozesszeit fortschreitende Belegung der Filterschicht mit dem abfiltrierten Kolloidmaterial erfolgt, wodurch sich die Filter langsam zusetzen. Außerdem entstehen in der auf der Filteroberfläche abgelagerten Kolloidschicht sehr hohe Partikelkonzentrationen, die eine unerwünschte und irreversible Partikel-Agglomeration erheblich begünstigen.

Auch das Abdestillieren von flüssigem Medium zur Erhöhung des Feststoffgehalts hat sich als nachteilig erwiesen, zum Einen, da es ein energieaufwendiges Verfahren darstellt, das unter erhöhter Temperatur und/oder mit reduziertem Druck erfolgen muß, zum Anderen, da die dispergierte schwerlösliche Verbindung durch die Temperaturbelastung möglicherweise geschädigt wird. Ein entscheidender Nachteil aller Verfahren, die auf einer Verdampfung von Flüssigkeit beruhen, ist außerdem, dass in diesem Fall nur die Flüssigkeit selbst, nicht aber die darin gelösten Stoffe entfernt werden. Geringe, nicht vermeidbare Verunreinigungen können daher im Endprodukt stark angereichert werden. Bei einer einprozentigen Feststoff-Konzentration in der Dispersion sind die Verunreinigungen in einem sprühgetrockneten Endprodukt um den Faktor 100 angereichert. Enthält die Dispersion verschiedene Dispersionsmittel bzw. Lösungsmittel, kann beim Eindampfen eine unterschiedlich schnelle Destillation eintreten, wodurch sich zwischenzeitlich Änderungen der Dispersionsmittel-/Lösungsmittelzusammensetzung ergeben, die für die Stabilität der kolloidalen Dispersion der schwerlöslichen Verbindung nachteilig sein kann.

In der WO 96/35414 ist in den Beispielen ein Verfahren zur Herstellung von Nanopartikeln eines schwer löslichen Wirkstoffs unter Einsatz einer cross-flow-Filtration beschrieben. Diese Filtration wird jedoch nicht zur Aufkonzentrierung sondern zur Reinigung der Dispersion unter erheblicher Volumenzunahme herangezogen.

Die US 5,948,441 beschreibt die Herstellung von Liposomen bzw. Lipidpartikeln. Die Tangentialfiltration wird verwendet, um extraliposomales bzw. außerhalb der Lipidpartikel befindliches Material, wie Lösungsmittel oder nicht eingeschlossenen Wirkstoff, abzutrennen.

Die genannten Probleme machen deutlich, dass trotz der Vorteile der mit den bisher bekannten Verfahren beschriebenen Formulierungen eine wirtschaftliche Herstellung wasserdispergierbarer Trockenpulver schwerlöslicher Verbindungen im Produktionsmaßstab nicht gelingen kann.

Aufgabe der vorliegenden Erfindung ist es daher, ein Verfahren zur Herstellung wasserdispergierbarer Trockenpulver schwerlöslicher Verbindungen bereitzustellen, das die Nachteile des Stands der Technik vermeidet.

Das Verfahren sollte insbesondere so geführt werden können, dass längere Lagerzeiten instabiler oder verderblicher Lösungen oder Dispersionen der schwerlöslichen Verbindungen vermieden werden.

Das Verfahren sollte außerdem eine wirtschaftliche Produktion der wasserdispergierbaren Trockenpulver erlauben.

Überraschenderweise wurde nun gefunden, dass zur Aufkonzentrierung der Dispersionen, welche die schwerlöslichen Verbindungen in kolloid-disperser Form enthalten, unter den geforderten Randbedingungen Wirtschaftlichkeit, kurze Prozesszeiten, Vermeidung von mikrobiellem Befall und Vermeidung von Agglomeration, das Verfahren der Tangentialfiltration oder "cross-flow-Filtration" besonders geeignet ist. Bei Verwendung dieses Verfahrens werden alle Nachteile von DestillationsVerfahren und weitgehend auch die Nachteile von Diafiltrations-Verfahren vermieden.

Die Erfindung betrifft daher ein Verfahren zur Herstellung wasserdispergierbarer Trockenpulver schwer wasserlöslicher Verbindungen, das folgende Schritte umfasst:
a) Herstellen einer Dispersion, die 0,5 bis 3 Gew.-% der schwerlöslichen Verbindung in mikrodisperser Form sowie ein Schutzkolloid in einem Dispersionsmittel enthält,
b) Aufkonzentrieren der Dispersion der schwerlöslichen Verbindung auf den 10- bis 40 fachen Feststoffgehalt durch Tangential-Filtration wobei eine aufkonzentrierte Dispersion erhalten wird, und
c) Entfernen des restlichen Dispersionsmittels aus der aufkonzentrierten Dispersion.

Durch das Aufkonzentrieren der Dispersion vor dem Entfernen des Dispersionsmittels wird erreicht, dass die Menge an Dispersionsmittel, die zeit- und energieaufwendig entfernt werden muss, verringert wird. Dadurch verkürzt sich der Zeitaufwand für die Dispersionsmittel-Entfernung derart, dass hergestellte Dispersionen ohne Zwischenlagerung sofort getrocknet werden können, ohne dass dabei unwirtschaftlich groß dimensionierte Trocknungsvorrichtungen nötig wären.

Schritt a) des erfindungsgemäßen Verfahrens, das Herstellen einer Dispersion, die die schwerlösliche Verbindung in mikrodisperser Form in einem Dispersionsmittel enthält, kann grundsätzlich in beliebiger Weise durchgeführt werden. Es sind zahlreiche Verfahren zur Herstellung einer derartigen Dispersion beschrieben, siehe den eingangs zitierten Stand der Technik. Es ist jedoch bevorzugt, die Dispersion nach dem Verfahren der sog. Mischkammer-Mikronisierung herzustellen, wie z.B. in der EP 0 065 193 A2 oder in der DE 37 02 030 A1 beschrieben ist. Der Offenbarungsgehalt dieser Anmeldungen, insbesondere hinsichtlich Verfahrensführung, hinsichtlich der verwendeten Lösungs- bzw. Dispersionsmittel, der Schutzkolloide und sonstiger Zusätze sowie hinsichtlich der Konzentrationen und Verhältnisse der verwendeten Verbindungen zueinander, wird hiermit durch Bezugnahme zum Inhalt der vorliegenden Erfindung gemacht. Demgemäß wird die Dispersion, die die schwerlösliche Verbindung in mikrodisperser Form enthält, erfindungsgemäß bevorzugt hergestellt, indem man die schwerlösliche Verbindung in einem flüchtigen, mit Wasser mischbaren, organischen Lösungsmittel bei Temperaturen zwischen 50 und 200°C, gegebenenfalls unter erhöhtem Druck, innerhalb einer Zeit von weniger als 10 s löst, und aus der erhaltenen molekulardispersen Lösung sofort durch schnelles Mischen mit einer wässrigen Lösung eines quellbaren Kolloids bei Temperaturen zwischen 0°C und 50°C die schwerlösliche Verbindung in kolloid-disperser Form ausfällt. In diesem Fall liegt also die schwerlösliche Verbindung in Form mikrodisperser Teilchen in einem Dispersionsmittel vor, das aus dem flüchtigen, mit Wasser mischbaren, organischen Lösungsmittel und aus Wasser besteht.

Alternativ wird die Dispersion, die die schwerlösliche Verbindung in mikrodisperser Form enthält, bevorzugt hergestellt, indem man die schwerlösliche Verbindung in einem flüchtigen, mit Wasser mischbaren, organischen Lösungsmittel bei Temperaturen zwischen 50 und 240°C, gemeinsam mit der 1,5- bis 20-fachen Gewichtsmenge, bezogen auf die schwerlösliche Verbindung, eines eßbaren Öls, sowie einem Emulgator, gegebenenfalls unter erhöhtem Druck, rasch löst, und aus der erhaltenen molekulardispersen Lösung durch sofortiges Mischen mit einer wässrigen Lösung eines Schutzkolloids bei Temperaturen zwischen 0°C und 50°C, die hydrophile Lösungsmittelkomponente in die wässrige Phase überführt, wobei die hydrophobe Ölphase, die die schwerlösliche Verbindung gelöst enthält, als mikrodisperse Phase entsteht. In diesem Fall ist die Dispersion also eine Zweiphasen-Mischung mit Ölteilchen als mikrodispersen Teilchen. Die schwerlösliche Verbindung ist in den Ölteilchen gelöst enthalten. Das Dispersionsmittel besteht aus dem flüchtigen, mit Wasser mischbaren, organischen Lösungsmittel sowie Wasser.

Als wassermischbare flüchtige Lösungsmittel sind Alkohole, Ketone, Ester, Acetale und Ether, insbesondere Aceton, 1,2-Butandiol-1-methylether, 1,2-Propandiol-1-n-propylether, Ethanol, n-Propanol, iso-Propanol und deren Gemische, bevorzugt.

Als Schutzkolloide kommen beliebige, für den Verwendungszweck zugelassene Schutzkolloide in Betracht, beispielsweise Gelatine, Stärke, Dextran, Pectin, Gummiarabicum, Casein, Caseinat, Vollmilch, Magermilch, Milchpulver oder Mischungen davon. Auch Polyvinylalkohol, Polyvinylpyrrolidon, Methylcellulose, Carboxymethylcellulose, Hydroxypropylcellulose und Alginate sind bevorzugte Kolloide.

Zusätzlich können zur Erhöhung der mechanischen Stabilität des Endprodukts dem Kolloid Weichmacher zugesetzt werden, beispielsweise Zucker oder Zuckeralkohole. Außerdem können je nach Bedarf, Konservierungsstoffe und/oder Oxidationsstabilisatoren zugesetzt werden. Geeignete Verbindungen sind jeweils in den oben genannten Patentanmeldungen angeführt. Als eßbare Öle, kommen insbesondere Öle in Betracht, die bei 20 bis 40°C flüssig sind. Beispiele sind pflanzliche Öle wie Maisöl, Kokosöl, Sesamöl, Arachisöl, Sojabohnenöl, Erdnußöl oder Baumwollsamenöl. Andere geeignete Öle oder Fette sind Schweineschmalz, Rindertalg und Butterfett. Die eßbaren Öle werden im Allgemeinen in der 1,5- bis 20-fachen, vorzugsweise 3- bis 8-fachen Gewichtsmenge, bezogen auf die schwerlösliche Verbindung angewandt, wobei der Gesamtölgehalt der Zubereitung der schwerlöslichen Verbindung 60 Gew.-% nicht überschreiten sollte, wenn ein Trockenpulver hergestellt werden soll.

Eine geeignete Vorrichtung zur Herstellung der Dispersionen ist ebenfalls in der EP 065 193 A2 und der DE 37 02 030 A1 beschrieben.

Im Allgemeinen weisen die Teilchen der Dispersion gemäß Stufe a) eine Größe im Bereich von 0,01 bis 100 µm, insbesondere 0,02 bis 10 µm auf. Besonders bevorzugt sind Dispersionen, in denen die dispergierten Teilchen mittlere Teilchengrößen von 0,01 bis 5 µm, bevorzugt 0,05 bis 0,8 µm besitzen. Diese sind beispielsweise gemäß EP 065 193, EP 239 949, EP 425 892 oder DE 37 02 030 erhältlich. Wenn die schwerlösliche Verbindung als solche kolloidal dispergiert ist, sind die dispergierten Teilchen in der Regel kleiner als wenn die schwerlösliche Verbindung in dispergierten Öltröpfchen gelöst ist. Das erfindungsgemäße Verfahren ist jedoch nicht auf Verbindungen mit diesen Teilchengrößen beschränkt.

Bei den schwer wasserlöslichen Verbindungen handelt es sich vorzugsweise um solche, die eine Löslichkeit von ≤ 10 g/l, insbesondere ≤ 5 g/l und besonders bevorzugt ≤ 1 g/l Wasser (bei 25°C) aufweisen.

Schwer wasserlösliche Verbindungen können organische oder anorganische Verbindungen sein. Bevorzugt sind pharmazeutische, diätische, kosmetische und pestizide Wirkstoffe, wobei keinerlei Einschränkung hinsichtlich des chemischen Typs besteht. Zu pharmazeutischen Wirkstoffen zählen auch Hormone, Vitamine, Provitamine, Enzyme, Phytopharmaka und Pflanzenextrakte. Beispiele für bevorzugte Wirkstoffgruppen und Wirkstoffe sind:
- Analgetika/Antirheumatika wie Codein, Diclofenac, Fentanyl, Hydromorphon, Ibuprofen, Indomethacin, Levomethadon, Morphin, Naproxen, Pritramid, Piroxicam, Tramadol
- Antiallergika wie Astemizol, Dimetinden, Doxylamin, Loratadin, Meclozin, Pheniramin, Terfenadin
- Antibiotika/Chemotherapeutika wie Erythromycin, Framycetin, Fusidinsäure, Rifampicin, Tetracyclin, Thiazetazon, Tyrothricin
- Antiepileptika wie Carbamazepim, Clonazepam, Mesuximid, Phenytoin, Valproinsäure
- Antimyotika wie Clotrimazol, Fluconazol, Itraconazol
- Calcium-Antagonisten wie Darodipin, Isradipin
- Corticoide wie Aldosteron, Betametason, Budesonid, Dexametason, Fluocortolon, Fludrocortison, Hydroxycortison, Methylprednisolon, Prednisolon
- Hypnotika/Sedativa
   Benzodiazepine, Cyclobarbital, Methaqualon, Phenobarbital
- Immunsuppressiva
   Azathioprin, Cyclosporin
- Lokalanaesthetika
   Benzocain, Butanilacain, Etidocain, Lidocain, Oxybuprocain, Tetracain
- Migränemittel
   Dihydroergotamin, Ergotamin, Lisurid, Methysergid
- Narkotika
   Droperidol, Etomidat, Fentanyl, Ketamin, Methohexital, Propofol, Thiopental
- Ophthalmika
   Acetazolamid, Betaxolol, Bupranolol, Carbachol, Carteolol, Cyclodrin, Cyclopentolat, Diclofenamid, Edoxodin, Homatropin, Levobununol, Pholedrin, Pindolol, Timolol, Tropicamid
- Phytopharmaka
   Hypernicum, Urtica folia, Artischocke, Agnus Castus, Cimicifuga, Teufelskralle, Besenginster, Pfefferminzöl, Eukalyptus, Schöllkraut, Efeu, Kava-Kava, Echinacea, Baldrian, Sabalextrakt, Hypericum, Mariendistel, Ginkgo Biloba, Aloe barbadensis, Allium sativum, Panax Ginseng, Serenoa Repens, Hydrastis canadensis, Vaccinium macrocarpon oder Mischungen daraus
- Proteasehemmer
   z.B. Saquinavir, Indinavir, Ritonavir, Nelfinavir, Palinavir, Tipranavir oder Kombinationen aus diesen Proteaseinhibitoren
- Sexualhormone und ihre Antagonisten
   Anabolika, Androgene, Antiandrogene, Estradiole, Gestagene, Progesteron, Oestrogene, Antioestrogene wie Tamoxifen
- Vitamine, Provitamine, Antioxidantien wie Carotinoide oder Carotinoid-Analoge, z.B. β-Carotin, Canthaxanthin, Astaxanthin, Lycopin oder Liponsäure, Vitamin A, Vitamin Q
- Zytostatika/Antimetastatika
   Busulfan, Carmustin, Chlorambucil, Cyclophosphamid, Dacarbacin, Dactinomycin, Estramustin, Etoposid, Flurouracil, Ifosfamid, Methotrexat, Paclitaxel, Vinblastin, Vincristin, Vindesin.

Die in Schritt a) erhaltene Dispersion wird erfindungsgemäß durch Tangentialfiltration aufkonzentriert (Schritt b)), wobei der Feststoff-Gehalt nach der Aufkonzentrierung bevorzugt 1 bis 20 Gew.-% beträgt. Tangentialfiltration ist ein an sich bekanntes Siebfiltrationsverfahren, bei dem im Gegensatz zur Diafiltration das zu filtrierende Medium nicht direkt auf die Filterschicht gepreßt wird, um dort einen Filterkuchen zu bilden, sondern in ständiger Bewegung gehalten wird. Wegen der kontinuierlichen Bewegung des zu filtrierenden Mediums spricht man auch von dynamischer Filtration. Eine Filterkuchen-Bildung wird verhindert oder zumindest stark verzögert, da das Filtermedium, d.h. die Filtrationsfläche, kontinuierlich freigespült wird. Die Bewegung des zu filtrierenden Mediums kann durch ständiges Umpumpen dieses Mediums erreicht werden, oder es kann ein Filter verwendet werden, der so ausgelegt ist, dass ihn das zu filtrierende Medium kontinuierlich durchströmen kann und auf seinem Weg durch den Filter vollständig oder in ausreichendem Maß von flüssigem Medium befreit wird.

Der Filtrationsvorgang findet an Membranen statt, deren Porengrößen entsprechend den Teilchengrößen der abzutrennenden Partikel auszuwählen sind. Bei abzutrennenden Partikeln einer Teilchengröße von etwa 0,01 µm bis etwa 0,1 µm spricht man von Ultrafiltration, bei abzutrennenden Partikeln einer Teilchengröße von etwa 0,1 µm bis etwa 10 µm von Mikrofiltration. Das Verfahren ist also gut geeignet, kolloid-disperse Partikel zurückzuhalten, d.h. Kolloid-Dispersionen aufzukonzentrieren.

Die Membranen zur Mikrofiltration und Ultrafiltration werden im Allgemeinen aus mechanischen Gründen auf einer ein- oder mehrschichtigen Unterstruktur als Träger aus dem gleichen oder unterschiedlichen Material wie die Membran aufgebracht. Die Trennschichten können aus organischen Polymeren, Keramik, Metall oder Kohlenstoff bestehen. Die Membranen werden in der Praxis in sogenannte Membranmodule eingebaut. Dabei kommen alle Modulgeometrien in Betracht, die unter den Temperatur- und Druckbedingungen der Filtration mechanisch beständig sind. Geeignet sind beispielsweise Flach-, Rohr-, Multikanalelement-, Kapillar- oder Wickelgeometrie.

Zur Erhöhung der Effizienz der Filtration wird die Tangentialfiltration üblicherweise als Druckfiltration betrieben, wobei der Druck typischerweise im Bereich von 0,2 bis 1 MPa liegt. Die Strömungsgeschwindigkeiten liegen typischerweise bei etwa 2 bis 4 m/s, und die Durchflußgeschwindigkeiten können, abhängig von Porengröße und Filtrationsdruck bis zu 3000 1 pro m² Filtermembran und Stunde betragen.

Das Aufkonzentrieren gemäß Schritt b) stellt einen Schritt in einem Gesamtverfahren dar und es ist daher wünschenswert, dass die dafür erforderlichen Prozesszeiten reproduzierbar und zuverlässig absehbar sind. Bei konventionellen Filtrationsprozessen treten Unwägbarkeiten auf, da die Filtrationsgeschwindigkeit durch die Bildung eines Filterkuchens und das Verstopfen der Filterporen mehr oder weniger stark abnimmt. Bei der Tangentialfiltration hingegen bleibt die durch die Membran abgeschiedene Flüssigkeitsmenge über die Prozesszeit weitgehend konstant und dem Verblocken der Filterporen wird ebenfalls entgegengewirkt. Weitere Vorteile sind, dass der Prozess sehr schonend durchgeführt werden kann, wodurch einem möglichen Partikelwachstum entgegengewirkt wird. Außerdem kann in geschlossenen Anlagen, falls erforderlich sogar keimfrei, gearbeitet werden, was im Hinblick auf die für mikrobiellen Befall anfälligen Schutzkolloide wünschenswert sein kann.

Es hat sich gezeigt, dass bei der vorliegenden Erfindung zur Aufkonzentrierung der Kolloid-Dispersionen insbesondere Membranen aus Polyethersulfon oder regenerierter Cellulose geeignet sind, wie sie z.B. von der Fa. Millipore unter der Bezeichnung BIOMAX (Polyethersulfon) und ULTRACELL angeboten werden. Verwendbar sind aber gleichermaßen auch Membranen anderer Hersteller und aus anderen Materialien hergestellte Membranen, z.B. solche, wie sie typischerweise zur Ultrafiltration eingesetzt werden. Die Filtermembranen werden in unterschiedlichen Filter-Porengrößen angeboten. Zur Aufkonzentrierung nach dem erfindungsgemäßen Verfahren kommen daher insbesondere solche Filtermembranen in Frage, die eine Molekulargewichts-Ausschlußgrenze von ab etwa MW 100 000 besitzen, d.h. Partikel oberhalb dieses Molekulargewichts werden von der Membran zurückgehalten und verbleiben in der aufkonzentrierten Kolloid-Dispersion, d.h. im Retentat. Bevorzugt sind Membranen mit MW-Ausschlußgrenzen von 500 000 bis 1 000 000.

Die Tangentialfiltration kann sehr spezifisch auf die jeweils aufzukonzentrierende Kolloidlösung eingestellt werden, da auf dem Markt eine Vielzahl unterschiedlicher Filtermembranen erhältlich sind, so dass praktisch jede gewünschte Filter-Porengröße und jedes gewünschte Filtermaterial zur Verfügung stehen. Die Filtermembranen sind standardisiert und in gleichbleibender Qualität erhältlich. Die Membranen werden als gebrauchsfertige Filtrationseinheit kommerziell angeboten, d.h. die Filtermembran ist in einem Metall- oder Kunststoffgehäuse eingebaut, das sowohl Anschlüsse für die aufzukonzentrierende Kolloidlösung besitzt als auch einen Auslass für die abfiltrierte Flüssigkeit (Filtrat). Entsprechende Komplett-Apparaturen werden vom Labor- bis zum Produktionsmaßstab kommerziell angeboten, angepaßt an die jeweiligen Aufgaben.

Eine besondere Ausführungsform der vorliegenden Erfindung ist die Kombination der Aufkonzentrierung der Kolloid-Dispersionen durch Tangential-Filtration mit Maßnahmen zur reversiblen Vergrößerung der Kolloidteilchen. Je größer der Molekulargewichtsunterschied zwischen abzutrennenden Bestandteilen und zurückzuhaltenden Partikeln ist, desto problemloser können sie voneinander getrennt werden. Es ist daher vorteilhaft, die in kolloidaler Form vorliegenden schwerlöslichen Verbindungen vor der Tangential-Filtration reversibel zu größeren Aggregaten zusammenzulagern. Dann können Filtermembranen mit größeren Porendurchmessern gewählt werden, wodurch die Filtrationsgeschwindigkeit erheblich steigt.

Eine reversible Agglomeration der Kolloidteilchen kann mit verschiedenen Verfahren erfolgen, z.B. durch die Zugabe von anorganischen und/oder organischen Salzen ("Aussalzen"), durch Temperaturerhöhung oder -erniedrigung, oder durch eine Änderung des pH-Wertes der Kolloid-Dispersion. Auch Kombinationen dieser Verfahren sind möglich.

Auf diese Weise können durch die reversible Agglomeration aus den ursprünglichen Kolloidteilchen, die bevorzugt im Größenbereich von etwa 50 bis 800 nm liegen, Aggregate im Größenbereich von Mikrometern bis Millimetern gebildet werden. Zur Aufkonzentrierung reichen dann sehr grobporige Membranen aus, die eine hohe Filtrationsgeschwindigkeit aufweisen.

Die Agglomeration muss reversibel sein, d.h. die ursprüngliche Teilchengrößen-Verteilung der schwerlöslichen Verbindungen in der Kolloid-Dispersion vor der Agglomeration muss sich wieder herstellen lassen. Welches der oben genannten Verfahren geeignet ist, kann im Einzelfall leicht durch Routineexperimente festgestellt werden. Bei der Verwendung ionischer Schutzkolloide, wie z.B. Casein bietet sich die Änderung des pH-Wertes an. Dieses anionische Schutzkolloid ist nur bei neutralen und schwach basischen pH-Werten löslich bzw. kolloidal löslich. Im sauren pH-Milieu findet eine Protonierung der Carboxylfunktion des Caseins statt, wodurch eine Ausfällung/Ausflockung erfolgt. Dieser Vorgang ist durch Erhöhung des pH-Wertes reversibel. Zubereitungen schwerlöslicher Verbindungen, die unter Verwendung von Casein als Schutzkolloid hergestellt wurden, lassen sich daher durch Absenken des pH-Wertes leicht ausfällen und können in diesem Zustand sehr effizient, d.h. schnell, aufkonzentriert werden. Nach Abtrennung der gewünschten Menge Lösungsmittel kann dann wieder eine pH-Wert-Erhöhung erfolgen, wodurch die ursprüngliche kolloidale Dispersion wieder erhalten wird.

Im Fall nichtionischer Schutzkolloide sind zur reversiblen Agglomeration andere Verfahren bevorzugt, z.B. die Zugabe konzentrierter Salzlösungen oder die Zugabe eines wasserlöslichen Salzes selbst.

Verfahren zur Agglomeration von Kolloid-Dispersionen sind Stand der Technik und lediglich im Einzelfall auf ihre Reversibilität zu überprüfen. Nach der Redispergierung der agglomerierten Partikel kann die Dispersion dann getrocknet werden.

Bei dem erfindungsgemäßen Verfahren können längere Standzeiten der Kolloid-Dispersionen vor der Trocknung vermieden werden, indem die Durchsatzleistung der Tangentialfiltrationseinheit an die pro Zeiteinheit anfallende Menge an Kolloid-Dispersion angepasst wird. Es ist möglich, die anfallende Menge an Kolloid-Dispersion ohne Zwischenlagerung direkt aufzukonzentrieren und ohne weitere Zwischenlagerung der Trocknung zuzuführen. Dies ist insbesondere dann vorteilhaft, wenn die Dispersion nach dem Aufkonzentrieren oder bereits vorher nicht ausreichend lagerstabil ist.

Das erfindungsgemäße Verfahren kann diskontinuierlich, halbkontinuierlich oder kontinuierlich durchgeführt werden. Es kann sich somit um ein Verfahren handeln, bei dem eine Charge einer Ausgangs-Dispersion hergestellt wird, diese Charge unmittelbar nach der Herstellung aufkonzentriert wird, und die aufkonzentrierte Charge unmittelbar nach Erreichen der gewünschten Konzentration vom Dispersionsmittel befreit wird, d.h., die einzelnen Schritte des erfindungsgemäßen Verfahrens können diskontinuierlich durchgeführt werden. Alternativ können die einzelnen Schritte selbst kontinuierlich durchgeführt werden, d.h. es kann beispielsweise die Ausgangs-Dispersion kontinuierlich oder diskontinuierlich hergestellt und kontinuierlich einer Tangential-Filtrationseinheit und nach der gewünschten Aufkonzentrierung einer Trocknungsvorrichtung zugeführt werden. Dazu ist eine Tangential-Filtrationseinheit bevorzugt, die so ausgelegt ist, dass die erforderliche Aufkonzentrierung bei einmaligem Durchströmen der Filtrationseinheit erreicht wird.

Aus der aufkonzentrierten Dispersion kann ein Trockenpulver in üblicher Weise, z.B. gemäß den Angaben der DE-OS 25 34 091 durch Sprühtrocknen, Abtrennen der Teilchen oder Trocknen im Wirbelbett erfolgen. Das bevorzugte Trocknungsverfahren ist das Sprühtrocknen. Die aufkonzentrierte Dispersion kann ohne weitere Vorbehandlung, wie z.B. Abziehen von Lösungsmittel durch Destillation sprühgetrocknet werden, d.h., das gesamte noch vorhandene Dispersionsmittel wird im Sprühturm abgezogen. Am Boden des Sprühturms fällt das wasserdispergierbare Trockenpulver in der Regel bereits trocken und rieselfähig an. Gegebenenfalls kann es zweckmäßig sein, ein durch Sprühtrocknung nur teilweise getrocknetes Pulver in einem Wirbelbett vollständig zu trocknen.

Im Folgenden wird die vorliegende Erfindung durch Beispiele, die erläuternd, nicht beschränkend zu verstehen sind, näher beschrieben.

### Beispiel 1:

Es wurde ein wasserdispergierbares Trockenpulver hergestellt, das 35,7 Gew.-% Coenzym Q10 und 64,3 Gew.-% Casein enthielt.

Zuerst wurde eine wässrige, kolloidale Lösung der angegebenen Bestandteile mittels Mischkammer-Mikronisierung, wie sie in der EP-0 065 193 A2 beschrieben ist, hergestellt. Die kolloidale Lösung hatte (vor der Aufkonzentrierung) einen Wirkstoffgehalt an Coenzym Q10 von 0,6 Masse-% und eine Partikelgrößenverteilung mit einem Schwerpunkt bei etwa 200 nm, wobei alle Teilchen kleiner als 1 µm waren. Diese Verteilung lag auch nach 24-stündiger Lagerung der Lösung unverändert vor, d.h. die Lösung war relativ lagerstabil.

Diese kolloidale Lösung wurde mittels Tangential-Filtration aufkonzentriert, die Bedingungen waren wie folgt:

| **Startbedingungen** | |
|---|---|
| Ausgangsvolumen | ca. 2,5 l |
| Temperatur | Raumtemperatur |

| **Prozessbedingungen** | |
|---|---|
| Membran: Ultracel® (Millipore GmbH, Eschborn) 100 kD V-Screen, Fläche | 0,1 m² |
| Feeddruck | 0,6 bar |
| Retentatdruck | 0,2 bar |
| Trans-Channel Druckabfall (dP) (Funktion des Cross flow) | 0,4 bar |
| Trans-Membrandruck(TMP) | 0,4 bar |
| Cross flow | 14 l/min/m² |
| Fluß-Ausgangswert bei t₀ | 34 l/h/m² |
| Fluß-Endwert bei.t_{final} | 8 l/h/m² |
| Mittlerer Gesamtfluß | 16 l/h/m² |
| Gesamtzeit der Aufkonzentrierung (t₀ -> t_{final}) | 125 min |
| TMP und dP wurden während des gesamten Aufkonzentrierungsprozesses konstant gehalten. | |
| Temperatur | Raumtemperatur |
| Wirkstoffkonzentration im Eluat Dies bedeutet, dass nur sehr wenig Wirkstoff durch die Membran hindurch der kolloidalen Lösung entzogen wurde. | < 0,07 % (m/m) |

| **Eigenschaften des Konzentrats** | |
|---|---|
| Endvolumen | Ca. 0,25 l |
| Temperatur | Raumtemperatur |
| Wirkstoffkonzentration | 7,1 % (m/m) |
| Faktor der Auf konzentrierung | ca. 12 |

Die verwendete Membran ist leicht zu reinigen. Spülen mit 0,1 n NaOH (ca. 10 min) bei Raumtemperatur führte zu einer nahezu vollständigen Wiederherstellung des Ausgangszustands (92,7 % der ursprünglichen NWP = Normalized Water Permeability). Dies bedeutet, dass das Produkt wenig bis gar nicht in die Membran eindringt und nur ein relativ geringes Adsorptionsvermögen zur Membran zeigt.

Formulierung A lässt sich somit unter schonenden Bedingungen in relativ kurzer Prozesszeit um den Faktor 12 aufkonzentrieren, ohne dabei nennenswerte Produktverluste in Kauf nehmen zu müssen.

### Beispiel 2:

Es wurde ein wasserdispergierbares Trockenpulver der folgenden Zusammensetzung hergestellt:

| Bestandteil | Masse [% (w/w)] |
|---|---|
| β-Carotin | 11,0 |
| Ascorbylpalmitat | 1,0 |
| α-Tocopherol | 2,0 |
| Gelatine B100 | 5,0 |
| GelitaSol P (Gelatinehydrolysat) | 25,0 |
| Lactose | 52,0 |
| Wasser (Restfeuchte) | 4,0 |

Analog Beispiel 1 wurde eine wässrige, kolloidale Lösung, welche die obigen Bestandteile enthielt, hergestellt. Der Wirkstoffgehalt an β-Carotin (vor dem Aufkonzentrieren) betrug 1,1 Masse-%. Die Partikelgrößenverteilung war bimodal. Ein Teil der Partikel wies einen Durchmesser von kleiner 1 µm auf und der Schwerpunkt der Verteilung lag hier bei etwa 200 nm. Der andere Schwerpunkt der Partikelgrößenverteilung lag bei etwa 16 µm, wobei der Partikeldurchmesser kleiner 20 µm war. Diese Verteilung lag auch nach 24-stündiger Lagerung der Lösung unverändert vor, d.h. die Lösung war relativ lagerstabil.

Bedingungen der Aufkonzentrierung durch Tangentialfluss-Filtration:

| **Startbedingungen** | |
|---|---|
| Ausgangsvolumen | ca. 5,0 l |
| Temperatur | Raumtemperatur |

| **Prozessbedingungen** | |
|---|---|
| Membran: Ultracel® (Millipore GmbH, Eschborn) 100 kD V-Screen, Fläche | 0,1 m² |
| Feeddruck | 0,6 bar |
| Retentatdruck | 0,1 bar |
| Trans-Channel Druckabfall (dP) (Funktion des Cross flow) | 0,5 bar |
| Trans-Membrandruck (TMP) | 0,35 bar |
| Cross flow | 16 l/min/m² |
| Fluß-Ausgangswert bei t₀ | 97 l/h/m² |
| Fluß-Endwert bei t_{final} | 30 l/h/m² |
| Mittlerer Gesamtfluß | 77 l/h/m² |
| Gesamtzeit der Aufkonzentrierung (t₀ -> t_{final}) | 34 min |
| TMP und dP wurden während des gesamten Aufkonzentrierungsprozesses konstant gehalten. | |
| Temperatur | Raumtemperatur |
| Wirkstoffkonzentration im Eluat Dies bedeutet, dass nur sehr wenig Wirkstoff durch die Membran hindurch der kolloidalen Lösung entzogen wurde. | < 0,001 % (m/m) |

| **Eigenschaften des Konzentrats** | |
|---|---|
| Endvolumen | Ca. 0,25 l |
| Temperatur | Raumtemperatur |
| Wirkstoffkonzentration | 21,3 % (m/m) |
| Faktor der Aufkonzentrierung | ca. 20 |

### Partikelgrößenverteilung:

Nach dem Aufkonzentrieren konnten keine Veränderungen bei der Partikelgrößengrößenverteilung im Vergleich zum Zustand vor dem Aufkonzentrierungsprozess festgestellt werden.

Die Dispersion läßt sich somit unter schonenden Bedingungen in einer kurzen Prozesszeit um den Faktor 20 aufkonzentrieren, ohne dabei eine Schädigung zu erleiden oder nennenswerte Produktverluste in Kauf nehmen zu müssen. Aus 200 l Ausgangslösung können innerhalb von 3 Stunden 10 l Konzentrat hergestellt werden. Dafür wird eine Membranfläche von 1 m² benötigt. Die verwendete Membran ist leicht zu reinigen: Bereits alleiniges Spülen mit Wasser (ca. 10 min) bei Raumtemperatur führt zu einer nahezu vollständigen Wiederherstellung des Ausgangszustands (93,7 % der ursprünglichen NWP = Normalized Water Permeability). Dies bedeutet, dass das Produkt wenig bis gar nicht in die Membran eindringt und nur ein geringes Adsorptionsvermögen zur Membran zeigt.

### Beispiel 3:

Analog Beispiel 1 wurde mit dem Schutzkolloid Casein (65 Gew.-%) und dem Wirkstoff Coenzym Q10 (35 Gew.-%) eine gelbliche, kolloidale wirkstoffhaltige Lösung hergestellt, die einen Gesamt-Feststoffgehalt von 0,5 Gew.-% besaß. Diese lösung wurde dann durch schrittweise Zugabe von wässriger Salzsäure (2 mol/l) auf pH=1 angesäuert, wodurch die kolloidal gelösten Feststoffe vollständig ausflockten. Dieser Niederschlag konnte über Vakuumfiltration (Papierfilter-Membran oder Glasfritte) abgesaugt werden; das Filtrat war farblos. Dieser abgetrennte Niederschlag wurde anschließend wieder unter Rühren bei Raumtemperatur in verdünnter Natronlauge (0,1 mol/l) in einer Konzentration von 0,5 % Gesamt-Feststoffgehalt dispergiert, wodurch wieder eine gelbliche, kolloidale Lösung entstand. Diese alkalische kolloidale Lösung konnte anschließend ohne Ausflockung auf einen pH von 7 mit Hilfe kleiner Mengen Salzsäure eingestellt werden.

Die Partikelgrößen-Verteilungen der erhaltenen kolloidalen Lösungen wurden mit Hilfe eines Partikelgrössen-Messgeräts Malvern Mastersizer gemessen. Die Ausgangs-Lösung vor der Ansäuerung mit HCl zeigte einen Mittelwert der Partikelgrösse von 0,2 µm (90 % unter 0,4 µm) ; es waren keine Partikel über 1 µm erkennbar. Der Mittelwert der Partikelgrössen-Verteilung des mit HCl ausgefällten und in verdünnter NaOH wieder redispergierten Niederschlags lag bei 0,3 µm (90 % unter 0,5 µm), nur etwa 1,5 % der Partikel lagen über 1 Mikrometer.

## Patentansprüche

1. Verfahren zur Herstellung wasserdispergierbarer Trockenpulver schwer wasserlöslicher Verbindungen, das folgende Schritte umfasst:
a) Bereitstellen einer Dispersion, die 0,5 bis 3 Gew.-% der schwerlösliche Verbindung in mikrodisperser Form sowie ein Schutzkolloid in einem Dispersionsmittel enthält,
b) Aufkonzentrieren der Dispersion der schwerlöslichen Verbindung auf den 10 - bis 40-fachen Feststoffgehalt durch Tangentialfiltration, wobei eine aufkonzentrierte Dispersion erhalten wird, und
c) Entfernen des restlichen Dispersionsmittels aus der aufkonzentrierten Dispersion.

2. Verfahren nach Anspruch 1, bei dem ein Dispersionsmittel verwendet wird, das aus Wasser und einem flüchtigen, mit Wasser mischbaren, organischen Lösungsmittel besteht.

3. Verfahren nach einem der vorhergehenden Ansprüche, bei dem eine Dispersion hergestellt wird, in der die dispergierten Teilchen Teilchengrößen von 0,01 bis 5 µm, bevorzugt 0,05 bis 0,8 µm, aufweisen.

4. Verfahren nach einem der vorhergehenden Ansprüche, bei dem bei der Tangential-Filtration eine Filtermembran aus Polyethersulfon oder regenerierter Cellulose verwendet wird.

5. Verfahren nach einem der vorhergehenden Ansprüche, bei dem bei der Tangential-Filtration eine Filtermembran mit einer Molekulargewichts-Ausschlußgrenze ab 100 000, bevorzugt von 500 000 bis 1 000 000, verwendet wird.

6. Verfahren nach einem der vorhergehenden Ansprüche, bei dem das Entfernen des Dispersionsmittels durch Sprühtrocknen erfolgt.

7. Verfahren nach einem der vorhergehenden Ansprüche, bei dem das Herstellen der Dispersion, das Aufkonzentrieren der Dispersion und das Entfernen des Dispersionsmittels kontinuierlich erfolgen.

8. Verfahren nach einem der vorhergehenden Ansprüche, bei dem die in der Dispersion enthaltenen mikrodispergierten Teilchen vor dem Aufkonzentrieren reversibel agglomeriert werden und nach dem Aufkonzentrieren mikrodispergiert werden.

9. Verfahren nach Anspruch 8, bei dem das Agglomerieren der dispergierten Teilchen durch
- Zugabe von anorganischen und/oder organischen Salzen, und/oder
- Temperaturänderung der Dispersion, und/oder
- pH-Wert-Änderung der Dispersion erfolgt.

## Claims

1. Method for producing water-dispersible dry powders from poorly watersoluble compounds, comprising the following steps:
a) Preparing a dispersion which contains 0.5 to 3 wt.% of the poorly soluble compound in microdispersed form and a protective colloid in a dispersion agent,
b) Concentrating the dispersion of the poorly soluble compound to 10 to 40 times the solid content by tangential filtration, thereby obtaining a concentrated dispersion, and
c) Removing the remaining dispersion agent from the concentrated dispersion.

2. Method according to claim 1, wherein a dispersion agent is used which consists of water and a volatile, water-miscible organic solvent.

3. Method according to one of the preceding claims, wherein a dispersion is prepared in which the dispersed particles have particle sizes of 0.01 to 5 µm, preferably 0.05 to 0.8 µm.

4. Process according to one of the preceding claims, wherein a filter membrane of polyethersulphone or regenerated cellulose is used in the tangential filtration.

5. Method according to one of the preceding claims, wherein a filter membrane with a molecular weight exclusion limit above 100,000, preferably from 500,000 to 1,000,000, is used in the tangential filtration.

6. Method according to one of the preceding claims, wherein the dispersion agent is eliminated by spray drying.

7. Method according to one of the preceding claims, wherein the preparation of the dispersion, the concentration of the dispersion and the removal of the dispersion agent take place continuously.

8. Method according to one of the preceding claims, wherein the microdispersed particles contained in the dispersion are reversibly agglomerated before the concentration and microdispersed after the concentration.

9. Method according to claim 8, wherein the agglomeration of the dispersed particles is carried out by
- adding inorganic and/or organic salts, and/or
- changing the temperature of the dispersion, and/or
- changing the pH of the dispersion.

## Revendications

1. Procédé de production de poudres sèches dispersibles dans l'eau à partir de composés difficilement solubles dans l'eau, lequel comprend les étapes suivantes consistant à :
a) préparer une dispersion qui contient 0,5 à 3 % en poids du composé difficilement soluble sous forme microdispersée ainsi qu'un colloïde protecteur dans un milieu de dispersion,
b) concentrer la dispersion du composé difficilement soluble par filtration tangentielle à 10 à 40 fois la teneur en matières solides, une dispersion concentrée étant obtenue, et
c) l'élimination du milieu de dispersion restant de la dispersion concentrée.

2. Procédé selon la revendication 1, dans lequel un milieu de dispersion est utilisé, lequel est constitué d'eau et d'un diluant organique miscible dans l'eau et volatil.

3. Procédé selon l'une quelconque des revendications précédentes, dans lequel une dispersion est produite, dans laquelle les particules dispersées présentent une granulométrie allant de 0,01 à 5 µm, de préférence de 0,05 à 0,8 µm.

4. Procédé selon l'une quelconque des revendications précédentes, dans lequel on utilise, au cours de la filtration tangentielle, une membrane filtrante en polyéthersulfone ou en cellulose régénérée.

5. Procédé selon l'une quelconque des revendications précédentes, dans lequel, au cours de la filtration tangentielle, on utilise une membrane filtrante ayant une limite d'exclusion de masse moléculaire à partir de 100 000, de préférence allant de 500 000 à 1 000 000.

6. Procédé selon l'une quelconque des revendications précédentes, dans lequel l'élimination du milieu de dispersion s'effectue par séchage par pulvérisation.

7. Procédé selon l'une quelconque des revendications précédentes, dans lequel la production de la dispersion, la concentration de la dispersion et l'élimination du milieu de dispersion s'effectuent en continu.

8. Procédé selon l'une quelconque des revendications précédentes, dans lequel les particules microdispersées contenues dans la dispersion sont agglomérées de manière réversible avant la concentration et sont microdispersées après la concentration.

9. Procédé selon la revendication 8, dans lequel l'agglomération des particules dispersées s'effectue par :
- ajout de sels inorganiques et/ou organiques, et/ou
- modification de la température de la dispersion, et/ou
- modification de la valeur du pH de la dispersion.
